# EUROPÄISCHE PATENTSCHRIFT

(11) **EP 0 608 693 B1**
(45) Veröffentlichungstag und Bekanntmachung des Hinweises auf die Patenterteilung: **23.10.1996**
(21) Anmeldenummer: 94100229.7
(22) Anmeldetag: 10.01.1994
(51) Int. Cl.: C07D 475/04, C07C 251/16

(54) **Verfahren zur Herstellung von Folsäure**
Process for the preparation of folic acid
Procédé pour la préparation de l'acide folique

(30) Priorität: 26.01.1993 CH 214/93
(43) Veröffentlichungstag der Anmeldung: 03.08.1994
(73) Patentinhaber: F. HOFFMANN-LA ROCHE AG, 4002 Basel (CH)
(72) Erfinder: Wehrli, Christof, CH-4108 Witterswil (CH)
(74) Vertreter: Cottong, Norbert A.

(56) Entgegenhaltungen:
- US-A- 2 442 836
- US-A- 2 442 837
- US-A- 2 444 002
- US-A- 2 956 057
- JOURNAL OF THE AMERICAN CHEMICAL SOCIETY, Bd.70, Nr.1, 1948 Seiten 25 - 26 R.B. ANGIER ET AL. 'Synthesis of Pteroylglutamic Acid. III'
- CHEMISCHE BERICHTE, Bd.88, Nr.7, 1955 Seiten 939 - 962 B. EISTERT ET AL. 'Versuche mit Triose-Redukton'

## Beschreibung

Die vorliegende Erfindung betrifft ein neues Verfahren zur Herstellung von Folsäure.

Robert B. Angier et al. (JACS, 70 (1948), 25 sowie US-A-2 442 836) beschreiben die Herstellung von Folsäure unter Einsatz von halogenfreien Verbindungen durch Umsetzung von p-Aminobenzoyl-L-glutaminsäurediäthylester mit 2-Hydroxymalondialdehyd, Isolierung von p-(2,3-Dihydroxy-2-en-propylidenamino)-benzoesäurediäthylester und Umsetzung des Zwischenprodukts mit Triaminopyrimidinon. Bei diesem Syntheseweg, bei dem ein Imin als Zwischenprodukt isoliert wird, wird Folsäure in lediglich 12,6% Ausbeute erhalten.

Weitere Herstellungsmethoden sind beschrieben in: O. Isler, G. Brubacher, S. Ghisla, B. Kräutler, Vitamine II; G. Thieme Verlag Stuttgart; (1988).

Allen diesen Methoden ist eine niedrige Ausbeute an Folsäure gemeinsam.

Der vorliegenden Erfindung liegt nun die Aufgabe zugrunde, ein Verfahren zur Herstellung von Folsäure bereitzustellen, gemäss welchem die Folsäure, im Vergleich zu bekannten Verfahren, in wesentlich höherer Ausbeute erhalten wird.

Ueberraschenderweise wurde nun gefunden, dass durch Einsatz eines Diimins der allgemeinen Formel I, 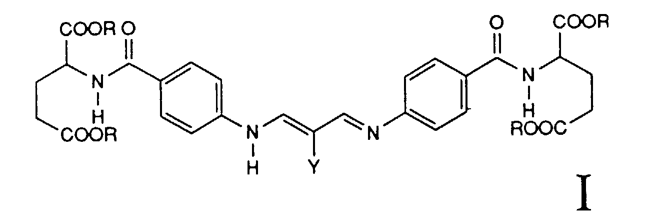 worin R Wasserstoff oder niederes Alkyl, Y OH, Halogen, Phosphat, Diphosphat, Triphosphat oder -OCOR' bedeuten, und R' niederes Alkyl oder Phenyl darstellt,
als Ausgangsprodukt Folsäure in wesentlich höheren Ausbeuten erhalten werden kann, und gleichzeitig die Entstehung von Nebenprodukten weitgehend vermieden werden kann.

Der Ausdruck "niederes Alkyl" bedeutet, im Rahmen der vorliegenden Erfindung, gradkettige oder verzweigte Alkylreste mit 1 bis 6 Kohlenstoffatomen, wie z.B. Methyl, Aethyl, Propyl, iso-Propyl, Butyl, iso-Butyl, tert.Butyl, Pentyl, Hexyl und dergleichen. Geradkettige Alkylreste, wie insbesondere Methyl und Aethyl, sind bevorzugt.

Der Ausdruck "Halogen" bedeutet, im Rahmen der vorliegenden Erfindung, insbesondere Chlor, Brom oder Jod. Bevorzugt ist Brom.

Bevorzugt werden Diimine der Formel I, worin R Wasserstoff und Y = OH ist, eingesetzt.

Aus B. Eistert et al. (Chem. Ber., 88 (1955), 939) sind Diimine bekannt, die durch Umsetzung von 2-Hydroxymalondialdehyd mit aromatischen Aminen wie Anilin, p-Aminobenzoesäure oder p-Aminobenzoesäureester hergestellt werden.

Die Diimine der obigen Formel I sind neue Verbindungen und ebenfalls Gegenstand der Erfindung.

Das erfindungsgemässe Verfahren ist nun dadurch gekennzeichnet, dass man ein Diimin der obigen Formel I mit Triaminopyrimidinon in Gegenwart von Sulfit, oder anorganischen Verbindungen, die in Wasser Sulfite bilden, bei einem pH-Wert von 3-8 und Temperaturen von etwa 0-100°C umsetzt.

Als Sulfite, oder anorganische Verbindungen, die in Wasser Sulfite bilden, werden Verbindungen wie z.B. Na₂SO₃, K₂SO₃, H₂SO₃, NaHSO₃, Na₂S₂O₅ oder SO₂ und dergleichen eingesetzt. Ebenso ist der Einsatz von Triaminopyrimidinon- Sulfit möglich.

Die Umsetzung der Diimine der allgemeinen Formel I zu Folsäure bzw. Folsäurealkylestern der Formel II erfolgt gemäss folgendem Reaktionsschema:

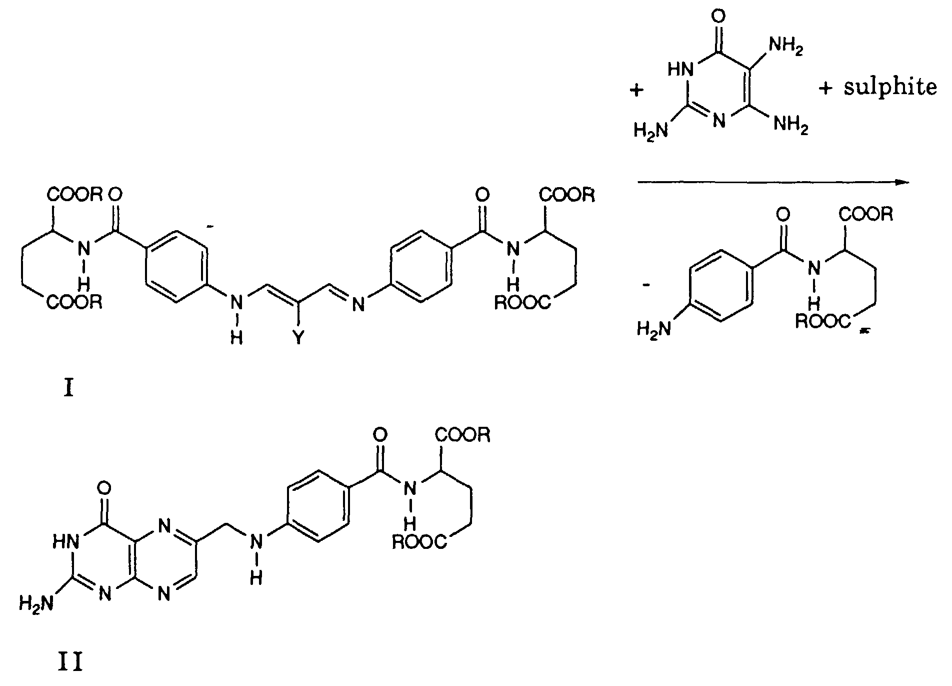

Die oben erwähnten Sulfite sind für die Umsetzung von Diiminen der Formel I zu Folsäure der Formel II essentiell. Ohne sie ist die Bildung von Nebenprodukten begünstigt.

Vorzugsweise wird mindestens etwa ein Aequivalent Triaminopyrimidinon in Gegenwart von 0,3-3 Aequivalenten Natriumsulfit bei einem pH-Wert von 5-7 bei 5-40°C mit Diiminen der Formel I umgesetzt. Besonders bevorzugt erweist sich der Einsatz von ca. 1 Aequivalent Natriumsulfit und eine Umsetzung von Triaminopyrimidon mit Diiminen der Formel I bei ca. 10°C. Die Umsetzung kann in wässriger Lösung oder auch in mit Wasser mischbaren, inerten, organischen Lösungsmitteln wie Acetonitril, Dimethylformamid, Dioxan, Methanol, Tetrahydrofuran und dergleichen erfolgen. Bevorzugt als Lösungsmittel ist Wasser. Falls andere Lösungsmittel verwendet werden, sollten diese mit Wasser vermischt sein und der Wasseranteil sollte grösser als 30% sein. Weiterhin erfolgt die Umsetzung zweckmässig bei einer Konzentration der Reaktanden von etwa 0,05-1 Mol/l, bevorzugt 0,1-0,5 Mol/l.

Die bei der erfindungsgemässen Herstellung der Folsäure als Ausgangsmaterial verwendeten Diimine der Formel I können in an sich bekannter Weise z. B. durch Umsetzung von 2-substituierten Malondialdehyden der Formel III 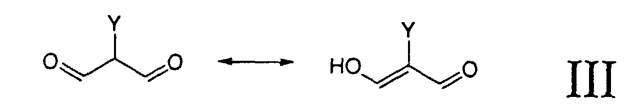 worin Y die in Formel I angegebene Bedeutung hat,
mit p-Aminobenzoyl-L-glutaminsäure bzw. p-Aminobenzoyl-L-glutaminsäureestern der Formel IV, 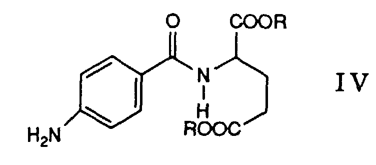 worin R die in Formel I angegebene Bedeutung hat,
unter sauren Bedingungen hergestellt werden. Hierbei können pro Aequivalent 2-substituierten Malondialdehyden der Formel III mindestens etwa 2 Aequivalente p-Aminobenzoyl-L-glutaminsäure bzw. p-Aminobenzoyl-L-glutaminsäureester der Formel IV eingesetzt werden. Die Umsetzung kann in wässrigem Medium, gegebenenfalls unter Zusatz von inerten, mit Wasser mischbaren, organischen Lösungsmitteln wie Acetonitril, Dimethylformamid, Dioxan, Methanol, Tetrahydrofuran und dergleichen erfolgen.

Die Umsetzung von 2-Hydroxymalondialdehyd mit p-Aminobenzoyl-L-glutaminsäure ist hierbei besonders bevorzugt und erfolgt zweckmässiger Weise bei pH-Werten kleiner als 4 und in einem Temperaturbereich von etwa 0°-60°C.

Anstelle von 2-substituierten Malondialdehyden der obigen Formel III kann auch von 2-substituierten Malondialdehydtetraalkylacetalen oder von 2-substituierten Malondialdehyddialkylhalbacetalen der Formel V ausgegangen werden, 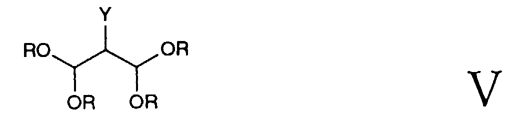 worin R die obige Bedeutung hat.

Unter sauren Hydrolysebedingungen entstehen aus den Verbindungen der Formel V in situ 2-substituierte Malondialdehyde der Formel III, aus denen die Diimine der Formel I dann gebildet werden können.

Besonders vorteilhaft hat sich ein ebenfalls neues Verfahren erwiesen, bei dem die saure Hydrolyse von Verbindungen der Formel V in Gegenwart eines Ionenaustauschers erfolgt. Ein geeigneter Ionenaustauscher ist im Rahmen der vorliegenden Erfindung z.B. DOWEX 50W.

Die bei der erfindungsgemässen Herstellung der Folsäure als Ausgangsmaterial verwendeten Diimine der Formel I können nach ihrer Herstellung isoliert und dann in dem erfindungsgemässen Verfahren eingesetzt werden. Sie können jedoch auch in situ hergestellt werden, und die Umsetzung kann demnach in einem Eintopfverfahren durchgeführt werden.

Werden als Ausgangsverbindungen Diimine der Formel I eingesetzt, worin R niederes Alkyl ist, so entstehen Folsäurealkylester, die in bekannter Weise hydrolisiert werden können, bevor die Folsäure isoliert wird.

Nach dem Abtrennen der Folsäure kann aus dem Filtrat p-Aminobenzoyl-L-glutaminsäure mit einem geeigneten Lösungsmittel wie z. B. Butanol, Methylacetat und dergleichen oder mit einem entsprechenden Lösungsmittelgemisch extrahiert und rezyklisiert werden.

Mittels des erfindungsgemässen Verfahrens können Ausbeuten an Folsäure von bis zu etwa 84% der Theorie erzielt werden.

Die folgenden Beispiele zeigen besonders vorteilhafte Ausführungsformen des erfindungsgemässen Verfahrens auf und sollen keinerlei Einschränkung darstellen.

### Beispiel 1:

### Herstellung von (S) - 2 - [4 - [3 - [4 - [(S) - 1,3 - dicarboxy - propylcarbamoyl] - phenylimino] - 2 - hydroxy - propenylamino] - benzoylamino] - pentan - 1,5 - di - Säure als Diimin der Formel I und Umsetzung des Diimins zur Folsäure.

In einem 100ml 5-Halskolben versehen mit Thermometer und Rührer werden unter Stickstoff 3.6 g (20 mMol) 1,1,3,3 -Tetramethoxy-2-propanol und 40 ml 1N HCl vorgelegt und 5 Minuten bei 50°C gerührt. Danach werden 10.65 g (40 mMol) p-Aminobenzoyl-L-glutaminsäure zugegeben und 1 Stunde bei 50°C gerührt. Nach Zugabe von ca. 200 ml Wasser lässt man das Diimin 24 Stunden bei Raumtemperatur kristallisieren, filtriert ab und wäscht den abfiltrierten Niederschlag mit Wasser. Nach dem Trocknen unter Wasserstrahlvakuum bei 60°C erhält man 10.1 g (S)-2-[4-[3-[4-[(S)-1,3-dicarboxy-propylcarbamoyl]-phenylimino]-2-hydroxy-propenylamino]-benzoylamino] - pentan - 1,5 - di - Säure mit einem Wassergehalt von 4%. Ausbeute = 83% d. Th.; Smp = 172-178°C.

Zu 6.1 g (S)-2-[4-[3-[4-[(S)-1,3-dicarboxy-propylcarbamoyl]-phenylimino]-2-hydroxy-propenylamino]-benzoylamino]-pentan-1,5-di-Säure mit einem Wassergehalt von 4% werden unter Stickstoff 20 ml Wasser und 2.52 g Natriumsulfit gegeben. Unter Rühren wird mit 2 m Natriumcarbonat-Lösung ein pH-Wert von 6.0 eingestellt. Zu der Suspension werden bei Raumtemperatur unter Rühren langsam 2.39 g (10 mMol) Triaminopyrimidinon-Sulfat zugegeben. Der pH-Wert wird durch Zudosieren von 2 m Natriumcarbonat-Lösung bei pH = 6.0 konstant gehalten. Nach 4 Stunden ist die Umsetzung beendet. In der rotbraunen Mischung wird die Folsäure bestimmt. Dazu wird die Reaktionsmischung mit ca. 220 ml Wasser auf genau 250 ml verdünnt und daraus ein aliquoter Teil entnommen zur Bestimmung der Folsäureausbeute mittels HPLC (Hochdruckflüssigkeitschromatographie). Ausbeute an Folsäure = 69% d.Th. bezogen auf eingesetztes Diimin.

### Beispiel 2:

### Herstellung von Folsäure durch Umsetzung von p-Aminobenzoyl-L-glutaminsäure, 2-Hydroxymalondialdehyd und Triaminopyrimidinon in wässrigem Medium (Eintopfverfahren)

In einem 100 ml 5-Halskolben versehen mit Thermometer und Rührer werden unter Stickstoff 5.32 g (20 mMol) p-Aminobenzoyl-L-glutaminsäure und 20 ml (2 mMol) 0.1N HCl vorgelegt. Dazu werden 0.88 g (10 mMol) 2-Hydroxymalondialdehyd gegeben und mit 3 ml Wasser nachgewaschen. Die Suspension wird 1 Stunde bei Raumtemperatur gerührt. Danach werden 4.25 ml 2 m Natriumcarbonat-Lösung zugegeben und ca. 20 Minuten weitergerührt. Danach werden 2.52 g (20 mMol) Natriumsulfit zugegeben und das Gemisch auf 38°C erwärmt. Zu der Suspension werden unter Rühren bei pH = 6.0 portionsweise im Verlauf von 1 Stunde 2.39 g (10 mMol) Triaminopyrimidinon-Sulfat zugegeben. Der pH-Wert wird durch Zudosieren von ca. 7.6 ml 2 m Natriumcarbonat-Lösung bei pH = 6.0 konstant gehalten. Nach 4 Stunden ist die Umsetzung beendet. In der rotbraunen Mischung wird die Folsäure, wie in Beispiel 1 beschrieben, bestimmt. Ausbeute an Folsäure = 68% d.Th. bezogen auf eingesetzten 2-Hydroxymalondialdehyd.

Zu Isolierung der Folsäure aus dieser Lösung wird der pH-Wert mit Essigsäure auf 3.0 eingestellt. Die Folsäure fällt aus und kann abfiltriert werden. Man erhält 3.08 g Folsäure - Rohprodukt mit einem Gehalt von 91% an reiner L-Folsäure = 63.5% d. Th.

Aus dem Filtrat wird die nicht umgesetzte p-Aminobenzoyl-L-glutaminsäure zurückgewonnen, indem mit Methylacetat bei pH = 3,0 erschöpfend extrahiert wird. Man erhält 3.23 g p-Aminobenzoyl-L-glutaminsäure-Rohprodukt mit einem Gehalt von 90% an reiner p-Aminobenzoyl-L-glutaminsäure. Der Gehalt an reiner p-Aminobenzoyl-L-glutaminsäure wird durch HPLC ermittelt.

### Beispiel 3:

### Herstellung von Folsäure durch Umsetzung von p-Aminobenzoyl-L-glutaminsäure, 2-Hydroxymalondialdehyd und Triaminopyrimidinon in wässrigem Medium unter Zusatz von organischen Lösungsmitteln.

In einem 100 ml 5-Halskolben versehen mit Thermometer und Rührer werden unter Stickstoff 5.32 g (20 mMol) p-Aminobenzoyl-L-glutaminsäure, 12.5 ml 0.2 N HCl, 12.5 ml Acetonitril und 0.88 g (10 mMol) 2-Hydroxymalondialdehyd gut gemischt und 24 Stunden bei Raumtemperatur stehen gelassen. Danach werden 2.52 g (20 mMol) Natriumsulfit zugegeben und das Gemisch auf 38°C erwärmt. Zu dieser Mischung werden unter Rühren bei pH = 6.0 langsam 2.39 g (10 mMol) Triaminopyrimidinon-Sulfat zugegeben. Der pH-Wert wird durch Zudosieren von 2 m Natriumcarbonat-Lösung bei pH = 6.0 konstant gehalten. Nach 6 Stunden ist die Umsetzung beendet. In der rotbraunen Mischung wird die Folsäure, wie in Beispiel 1 beschrieben, bestimmt. Die HPLC Analyse ergibt eine Folsäureausbeute von 73% d. Th.

### Beispiel 4:

### Herstellung von Folsäure durch Umsetzung von p-Aminobenzoyl-L-glutaminsäure, 2-Hydroxymalondialdehyd und Triaminopyrimidinon in wässrigem Medium unter Zusatz von organischen Lösungsmitteln.

In einem 100 ml 5-Halskolben versehen mit Thermometer und Rührer werden unter Stickstoff 5.32 g (20 mMol) p-Aminobenzoyl-L-glutaminsäure, 12.5 ml 0.2 N HCl, 12.5 ml Acetonitril und 0.88 g (10 mMol) 2-Hydroxymalondialdehyd gut gemischt und 24 Stunden bei Raumtemperatur stehen gelassen. Danach werden 1.26 g (10 mMol) Natriumsulfit und 9 ml 2m Natriumcarbonatlösung zugegeben und das Gemisch 1 Stunde bei 10°C gerührt. Zu dieser Mischung werden bei 10°C unter Rühren bei pH = 6.0 langsam 2.39 g (10 mMol) Triaminopyrimidinon-Sulfat zugegeben. Der pH-Wert wird durch Zudosieren von 2 m Natriumcarbonat-Lösung bei pH = 6.0 konstant gehalten. Nach ca. 3 Tagen ist die Umsetzung beendet. In der rotbraunen Mischung wird die Folsäure, wie in Beispiel 1 beschrieben, bestimmt. Die HPLC Analyse ergibt eine Folsäureausbeute von 84% d. Th.

### Beispiel 5:

### Herstellung von Folsäure durch Umsetzung von Triaminopyrimidinon mit (S) - 2 - [4 - [3 - [4 - [(S) - 1,3 - dicarboxy - propylcarbamoyl] - phenylimino] - 2 - hydroxy - propenylamino] - benzoylamino] - pentan - 1,5 - di - Säure in Wasser.

In einem 100ml 5-Halskolben versehen mit Thermometer und Rührer werden unter Stickstoff 2.23 g ( 10mMol) Triaminopyrimidinon - Sulfit, 1.26 g ( 10mMol) Natriumsulfit und 30 ml Wasser vorgelegt. Danach werden bei pH = 6.0 und 38°C unter Rühren langsam 6.1 g (S)-2-[4-[3-[4-[(S)-1,3-dicarboxy-propylcarbamoyl]-phenylimino]-2-hydroxy-propenylamino]-benzoylamino]-pentan-1,5-di-Säure ( Diimin aus Beispiel 1) zugegeben. Der pH-Wert wird durch Zudosieren von 10 ml 2 m Natriumcarbonat-Lösung bei pH = 6.0 konstant gehalten. Nach 6 Stunden ist die Umsetzung beendet. In der rotbraunen Mischung wird die Folsäure, wie in Beispiel 1 beschrieben, bestimmt. Die HPLC Analyse ergibt eine Folsäureausbeute von 64% d. Th.

### Beispiel 6:

### Herstellung von 2-Hydroxymalondialdehyd durch saure Hydrolyse von 2-Hydroxymalondialdehydtetramethylacetal in Gegenwart eines Ionenaustauschers

Eine Lösung von 34.2 g (190 mMol) 2-Hydroxymalondialdehydtetramethylacetal in 600 ml Wasser wird bei Raumtemperatur mit einem Durchfluss von 2.5 ml/Minute. durch eine mit ca. 130 g DOWEX 50W gefüllte Glassäule (260 x 25 mm) gegeben.

Das Eluat wird bei etwa 0°C unter Stickstoff aufgefangen und am Rotationsverdampfer im Vakuum (≤ 20 mbar) auf 50 g eingeengt. Die Suspension wird 18 Stunden bei 0°C stehen gelassen. Das Roh-Produkt wird abgenutscht und mit 10 ml Wasser nachgewaschen. Nach 4 Stunden Trocknen im Wasserstrahlvakuum bei 40°C erhält man 12.04 g 2-Hydroxymalondialdehyd. Smp. 157/8°C unter Zersetzung. Gehalt = 98% (jodometrische Titration).

Aus der Mutterlauge wird ein zweites Kristallisat erhalten.

1.36 g 2-Hydroxymalondialdehyd. Gehalt = 97% (jodometrische Titration). Gesamtausbeute: 13.40 g 2-Hydroxymalondialdehyd = 78% d.Th.

## Patentansprüche

1. Verfahren zur Herstellung von Folsäure, dadurch gekennzeichnet, dass man ein Diimine der Formel I 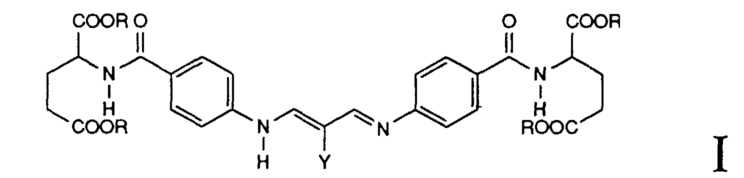 worin R Wasserstoff oder niederes Alkyl, Y OH, Halogen, Phosphat, Diphosphat, Triphosphat oder -OCOR' bedeuten und R' niederes Alkyl oder Phenyl darstellt,
mit Triaminopyrimidinon in Gegenwart von Sulfit, oder anorganischen Verbindungen, die in Wasser Sulfite bilden, bei einem pH-Wert von 3-8 und Temperaturen von etwa 0-100°C umsetzt.

2. Verfahren zur Herstellung von Folsäure gemäss Anspruch 1, dadurch gekennzeichnet, dass die Umsetzung mit Triaminopyrimidinon in Gegenwart von 0,3-3 Mol Aequivalent Natriumsulfit bei einem pH-Wert von 5-7 und Temperaturen von 5°-40°C erfolgt.

3. Verfahren zur Herstellung von Folsäure gemäss Anspruch 1 oder 2, dadurch gekennzeichnet, dass das Verfahren als Eintopfverfahren geführt wird.

4. Diimin der Formel I 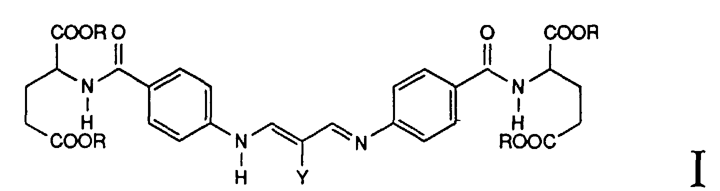 worin R Wasserstoff oder niederes Alkyl, Y OH, Halogen, Phosphat, Diphosphat, Triphosphat oder -OCOR' bedeuten, und R' niederes Alkyl oder Phenyl darstellt.

## Claims

1. A process for the manufacture of folic acid, characterized by reacting a diimine of formula I 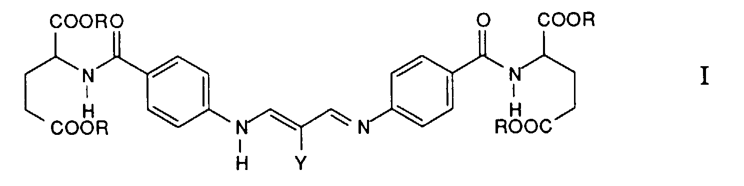 wherein R signifies hydrogen or lower alkyl, Y signifies OH, halogen, phosphate, diphosphate, triphosphate or -OCOR' and R' represents lower alkyl or phenyl,
with triaminopyrimidinone in the presence of sulphite, or inorganic compounds which form sulphites in water, at a pH value of 3-8 and temperatures of about 0-100°C.

2. A process for the manufacture of folic acid according to claim 1, characterized in that the reaction with triaminopyrimidinone is effected in the presence of 0.3-3 mol equivalents of sodium sulphite at a pH value of 5-7 and temperatures of 5-40°C.

3. A process for the manufacture of folic acid according to claim 1 or 2, characterized in that the process is carried out as a one-pot process.

4. A diimine of formula I 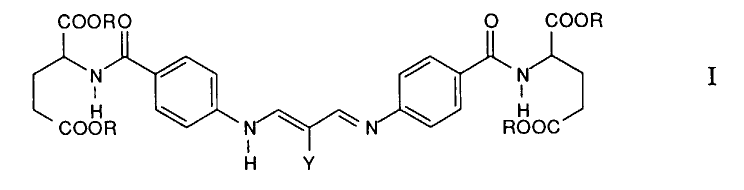 wherein R signifies hydrogen or lower alkyl, Y signifies OH, halogen, phosphate, diphosphate, triphosphate or -OCOR' and R' represents lower alkyl or phenyl.

## Revendications

1. Procédé pour la préparation de l'acide folique, caractérisé en ce que l'on fait réagir une diimine de formule I 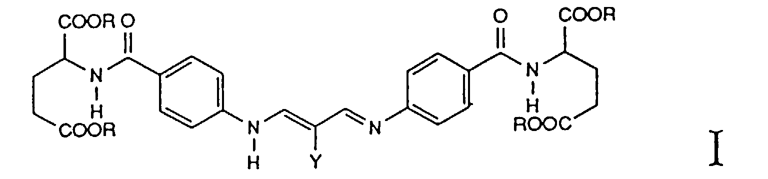 dans laquelle R représente un atome d'hydrogène ou un groupe alkyle inférieur, Y représente OH, un atome d'halogène ou un groupe phosphate, diphosphate, triphosphate ou -OCOR', et R' représente un groupe alkyle inférieur ou phényle,
avec de la triaminopyrimidinone en présence de sulfite, ou de composés minéraux qui forment dans l'eau des sulfites, à un pH de 3 à 8 et à des températures d'environ 0-100°C.

2. Procédé pour la préparation de l'acide folique selon la revendication 1, caractérisé en ce que la réaction avec la triaminopyrimidinone s'effectue en présence de 0,3 à 3 équivalents molaires de sulfite de sodium, à un pH de 5-7 et à des températures de 5 à 40°C.

3. Procédé pour la préparation de l'acide folique selon la revendication 1 ou 2, caractérisé en ce que le procédé est effectué en tant que procédé en un seul récipient.

4. Diimine de formule I 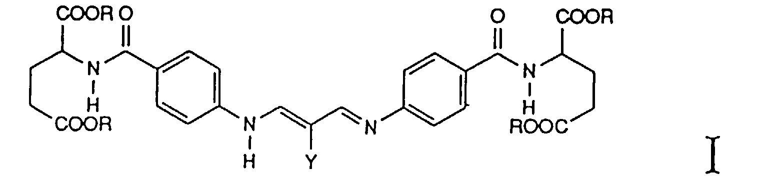 dans laquelle R représente un atome d'hydrogène ou un groupe alkyle inférieur, Y représente OH, un atome d'halogène ou un groupe phosphate, diphosphate, triphosphate ou -OCOR', et R' représente un groupe alkyle inférieur ou phényle.
